# EUROPÄISCHE PATENTSCHRIFT

(11) **EP 0 623 590 B1**
(45) Veröffentlichungstag und Bekanntmachung des Hinweises auf die Patenterteilung: **14.10.1998**
(21) Anmeldenummer: 94103294.8
(22) Anmeldetag: 04.03.1994
(51) Int. Cl.: C07C 253/30, C07C 255/17

(54) **Verfahren zur Herstellung von Alkalisalzen des Cyanacetons**
Process for the preparation of the alkali metal salts of cyanoacetone
Procédé de préparation de sels alcalins de cyanoacétone

(30) Priorität: 05.05.1993 DE 4314848
(43) Veröffentlichungstag der Anmeldung: 09.11.1994
(73) Patentinhaber: HÜLS AKTIENGESELLSCHAFT, 45764 Marl (DE)
(72) Erfinder: Muhr, Jürgen, Dr., D-53347 Alfter (DE); Feld, Marcel, Dr., D-51147 Köln (DE)

(56) Entgegenhaltungen:
- DE-A- 3 514 688
- 'Houben-Weyl Methoden der Organischen Chemie Bd. 8, S. 573-4' , E. MüLLER , STUTTGART 1952 & NATURWISSENSCHAFTEN Bd. 33 , 1946 Seiten 157 - 158 C. K. HAUSER, R. LEVINE 'Zur Kondensation von aliphatischen Nitrilen mit Estern, Aldehyden und Ketonen'
- COMPTES RENDUS HEBDOMAIRES DES SEANCE DE L'ACADEMIE DES SCIENCES Bd. 259, Nr. 6 , 1964 Seiten 1418 - 1421 H. GAULT, R. BLOCH 'Sur la condensation du diéthoxyacétate d'éthyle avec l'acétonitrile: diéthoxy-4.4 céto-3 butyronitrile'

## Beschreibung

Die Erfindung betrifft ein Verfahren zur Herstellung von Alkalisalzen des Cyanacetons durch Umsetzung von Acetonitril und einem Essigsäureester, dessen Alkoholkomponente ein substituierter oder nicht substituierter, geradkettiger, verzweigter oder cyclischer Alkohol ist in Gegenwart von Alkalialkoholat, dessen Alkoholkomponente ein substituierter oder nicht substituierter, geradkettiger, verzweigter oder cyclischer Alkohol ist bei Temperaturen von 40 bis 160 °C, **dadurch gekennzeichnet**, daß die Umsetzung ohne Abdestillation des wahrend der Reaktion gebildeten Alkohols durchgeführt wird.

Das Produkt Alkali-Cyanaceton mit der enolischen Struktur der Formel I, worin M Natrium oder Kalium ggf. Lithium bedeutet,

H₃C-C(OM) = CH (CN) Formel I

ist als Synthesebaustein zur Herstellung von Pharmawirkstoffen von Interesse.

Es ist bekannt, β-Ketonitrile durch Kondensation von Nitrilen mit aliphatischen oder araliphatischen Carbonsäureestern herzustellen (Houben-Weyl, Bd. 8, 573-4 (1952). Aliphatische Nitrile benötigen die Verwendung von Natriumamid als Kondensationsmittel und ein Abdestillieren des gebildeten Alkohols. Nach DE-OS 35 14 688 ist auch Natriummethylat statt Natriumamid oder Natriumhydrid verwendbar, wenn ein hoher Überschuß des Nitrils eingesetzt und der bei der Reaktion gebildete Alkohol zur Verschiebung des Gleichgewichts laufend entfernt wird. Dieser Weg setzt aber Carbonsäureester mit relativ hohen Siedepunkten voraus. Essigsäure-methyl- bzw. ethylester besitzen zu niedrige Siedepunkte und destillieren ab, wie auch Vergleichsbeispiel A zeigt. Andererseits sind gemäß Vergleichsbeispiel B nicht Essigsäureester längerkettiger Alkohole mit Erfolg einsetzbar, da diese umgeestert werden.

Es bestand somit die Aufgabe, ein einfach ausführbares Verfahren zur Herstellung von Alkalicyanaceton zu finden, bei dem die genannten Nachteile vermieden werden.

Diese Aufgabe wird durch das erfindungsgemäße Verfahren gelöst.

Gegenstand der Erfindung ist ein Verfahren zur Herstellung von Alkalisalzen des Cyanacetons durch Umsetzung von Acetonitril und einem Essigsäureester in Gegenwart von Alkalialkoholat bei erhöhter Temperatur, dadurch gekennzeichnet, daß die Umsetzung ohne Abdestillation des während der Reaktion gebildeten Alkohols durchgeführt wird.

Nach dem erfindungsgemäßen Verfahren werden Verbindungen der Formel I, bevorzugt Natriumcyanaceton (NaCyA) und Kaliumcyanaceton, hergestellt.

Als Essigsäureester eignen sich solche mit Alkoholkomponenten, deren Rest R ein substituierter oder nicht substituierter, geradkettiger, verzweigter oder cyclischer aliphatischer Rest mit 1 bis 20, vorzugsweise 1 bis 6 C-Atomen ist. Der Rest kann mit Alkyl-, Alkoxy-,(Poly) alkoxy, Halogen und/oder Arylresten substituiert sein. Bevorzugt sind Essigsäuremethylester und Essigsäureethylester. Andererseits können Essigsäureester mit 3 oder mehr C-Atomen im Alkoholrest, dann zusammen mit Alkoholaten dieser Alkohole, bevorzugt sein, die leichter von Acetonitril bei der Aufarbeitung durch Destillation trennbar sind.
Als Alkalialkoholate sind die mit Resten R, wie oben definiert, verwendbar. Bevorzugte Reste R sind die der aliphatischen Alkohole mit 1 bis 6 C-Atomen.

Acetonitril wird bevorzugt im Verhältnis von 1 bis 12 mol, sehr bevorzugt 1,5 bis 6 mol Acetonitril je mol Alkalialkoholat verwendet. Sehr bevorzugt wird Alkalialkoholat frei von Alkoholen als Lösungsmittel eingesetzt. Weiter bevorzugt wird Alkalialkoholat in Acetonitril suspendiert eingesetzt.

Weitere inerte Lösemittel sind verwendbar, werden jedoch vorzugsweise vermieden. Solche Lösungsmittel wie Ether, Polyethylenglykole, Polyethylenglykolether, N-Methypyrrolidon, Propylencarbonat oder Ethylenharnstoff können insbesondere in Mengen von 10 bis 30% positive Effekte auf die rheologischen Eigenschaften und Filtrierbarkeit bewirken. Auch bei Verzicht auf Alkohole als Lösungsmittel für die Alkoholate brauchen überraschend nicht fremde Lösemittel zugesetzt werden.

Bei der Wahl der Einsatzstoffe sind gleiche Alkoxy-Reste im Ester und Alkalialkoholat nicht zwingend, aber bevorzugt. So kann beispielsweise Natriumcyanaceton aus Acetonitril, Ethylacetat bzw. Methylacetat und Natriummethylat gewonnen werden. Gemische von Alkoholaten und Gemische von Essigsäureestern sind auch verwendbar. Nach dem Verfahren können die Einsatzstoffe gemeinsam erhitzt und zur Reaktion gebracht werden.
Nach dem Verfahren kann auch der Essigsäureester dem Acetonitril und dem Alkoholat, bevorzugt der Suspension daraus, bei Reaktionstemperatur zugegeben bzw. langsam zudosiert werden. Hier sind Zeiten von bis zu 10, vorzugsweise bis zu 2 St. zweckmäßig.
Es kann auch Essigsäureester und/oder eine Suspension aus Alkoholat und Acetonitril und/oder Acetonitril und Essigsäureester portionsweise oder kontinuierlich unter Reaktionsbedingungen zugegeben werden.
Um während der Aufheizphase Polymerisation von Acetonitril in Gegenwart von Alkalialkoholat zu unterdrücken, bietet es sich an, diese Phase zu verkürzen, indem man das Alkoholat in einem Teil des Nitrils suspendiert und zu dem auf Reaktionstemperatur erwärmten Nitril zufügt, bevor man mit der Dosierung des Essigsäureesters beginnt. Die Kondensationsreaktion geschieht vorzugsweise in Inertatmosphäre durch z.B. N₂ bei Temperaturen von 40 bis 160, sehr bevorzugt bei 80 bis 120°C, unter Eigendruck oder bei erhöhtem Druck von 2 bis 200, vorzugsweise 2 bis 20 bar.

Da erfindungsgemäß auf die Abdestillation des Alkohols verzichtet wird, läßt sich das Verfahren auch in einem Rührkessel oder Umlaufreaktoren oder in Rührkesselkaskaden durchführen. Überraschend entstehen geringe Verluste durch Eigenkondensation von Acetonitril und Essigsäureester, obwohl in den Vergleichsbeispielen A und B Eigenkondensation auftritt.
Diese Aufarbeitung kann bei z.B. 40 bis 60°C durch Filtration erfolgen, wobei allgemein kein Verdünnen mit z.B. Acetonitril oder einem Alkohol nötig ist.
Das Alkalisalz kann zu Cyanaceton z.B. mit Säuren oder CO₂ umgesetzt werden.

### Beispiel 1

In einem 1,5 l Autoklaven legt man im Stickstoffstrom 500,2 g (12,2 mol) Acetonitril und 135 g (2,5 mol) Na-Methylat vor und erhitzt die Suspension unter Rühren auf 90°C.
Nachdem der Reaktionsdruck mit N₂-Gas auf 3 atm eingestellt ist, dosiert man mit einer Druckpumpe 185 g (2,5 mol) Essigsäuremethylester in 4 St. ein. Man läßt noch 1/2 Stunde nachreagieren, kühlt auf 50°C ab, filtriert durch einen Druckfilter, wäscht mit Essigester, oder Acetonitril und trocknet im Vakuum.
Ausbeute: 183 g Natrium-Cyanaceton (Gehalt über 98% nach HLPC) = über 70% d. Th.

### Beispiel 2

In einem 1,5 l Autoklaven legt man unter N₂ 287 g (7 mol) Acetonitril vor, erhitzt unter Rühren auf 90°C und trägt eine Suspension von 135 g (2,5 mol) Na-Methylat in 213,2 g (5 mol) Acetonitril ein. Nachdem der Reaktionsdruck mit N₂-Gas auf 3 atm eingestellt ist, dosiert man mit einer Druckpumpe 185 g (2,5 mol) Essigsäuremethylester in 4 Std. ein. Man läßt 1/2 l Std. nachreagieren, kühlt auf 50°C ab und filtriert über einen Druckfilter und trocknet.
Ausbeute: 190 g NaCyA (Gehalt über 98%) = über 71% d.Th.

### Beispiel 3

In einem 1,5 l Autoklaven legt man unter N₂ 492,6 g (12 mol) Acetonitril und 204 g (3 mol) Na-Ethylat vor und erhitzt die Suspension unter Rühren auf 90°C. Nachdem der Reaktionsdruck mit N₂-Gas auf 3 atm eingestellt ist, dosiert man 352,4 g (4 mol) Essigsäureethylester in 4 St. ein. Man läßt 1/2 St. nachreagieren, kühlt auf 50°C ab und filtriert über einen Druckfilter und trocknet.
Ausbeute: NaCyA (Gehalt über 97%) = über 74% d. Th.

### Beispiel 4

In einem 1,5 l Autoklaven legt man 492,6 g (12 mol) Acetonitril und 162 g (3 mol) Natriummethylat vor, erwärmt auf 90°C und dosiert zu dieser Suspension in 4 St. 352,4 g (4 mol) Essigsäureethylester. Nach 30 Min. Nachreaktion wird bei 50°C abgelassen und wie in den vorangegangenen Beispielen aufgearbeitet.
Ausbeute: 234 g (Gehalt über 95%) = über 71% d. Th.

### Beispiel 5

In einem 2 l Autoklaven werden 986 g (24 mol) Acetonitril, 108 g ( 2 mol) Na-Methylat und 264 g (3 mol) Essigsäureethylester gemischt, in 2 St. auf 90°C erhitzt. Nach Abkühlen auf 50°C wird entleert, das Salz abfiltriert und im Vakuum getrocknet.
Ausbeute: 197,5 g (Gehalt über 88%) = über 83% d.Th.

### Vergleichsbeispiel A

In einer Rührapparatur mit Rührer, Tropftrichter, Gaseinleitungsrohr und aufgesetzter Destillationskolonne wurde unter Rühren und Einleiten von Stickstoff 74,1 g ( 1 mol) Essigsäuremethylester und 550 g (13,4 mol) Acetonitril zum Sieden erhitzt. In die siedende Reaktionslösung wurden in 2 St. 180 g 30 Gew.-%ige (1 mol) Natriummethylatlösung eingetropft. Gleichzeitig wurden die Leichtsieder aus der Natriummethylatlösung stammendes und entstehendes Methanol, Essigsäuremethylester und Acetonitril über die Destillationskolonne abdestilliert. Nach Ende der Zugabe der Methylatlösung wurde die Destillation noch weitere 3 Std. weitergeführt und insgesamt 269,8 g Destillat erhalten, das 61 g (0,82 mol) Methylacetat enthielt. Nach Abkühlen des Reaktionsgemisches wurde der Niederschlag abfiltriert und mit Aceton gewaschen.
Ausbeute: 81,5 g (20 Gew.%ig) = 15,5 % d.Th.

### Vergleichsbeispiel B

In der Rührapparatur wurden unter Rühren und Einleiten von N₂ 116,1 g ( 1 mol) Essigsäure-n-butylester und 550 g (13,4 mol) Acetonitril zum Sieden erhitzt. In die siedende Reaktionslösung wurden in 2 St. 180 g 30 Gew.-%ige (1 mol) Natriummethylatlösung eingetropft. Gleichzeitig wurde aus der Natriummethylatlösung stammendes Methanol, n-Butanol umgeesterter Essigsäuremethylester und Acetonitril abdestilliert. Nach Ende der Zugabe der Methylatlösung wurde die Destillation noch 3 St. weitergeführt und insgeamt 531 g Destillat erhalten, das 47,8 g (0,64 mol) Methylacetat enthielt. Nach Abkühlen des Reaktionsgemisches wurde das ausgefallene NaCyA abfiltriert und mit Aceton gewaschen.
Ausbeute: 32,8 g (84 Gew.-%ig) = 26% d.Th.

## Patentansprüche

1. Verfahren zur Herstellung von Alkalisalzen des Cyanacetons durch Umsetzung von Acetonitril und einem Essigsäureester, dessen Alkoholkomponente ein substituierter oder nicht substituierter, geradkettiger, verzweigter oder cyclischer Alkohol ist in Gegenwart von Alkalialkoholat, dessen Alkoholkomponente ein substituierter oder nicht substituierter, geradkettiger, verzweigter oder cyclischer Alkohol ist bei Temperaturen von 40 bis 160 °C, **dadurch gekennzeichnet**, daß die Umsetzung ohne Abdestillation des wahrend der Reaktion gebildeten Alkohols durchgeführt wird.

2. Verfahren nach Anspruch 1, **dadurch gekennzeichnet,** daß der Essigsäureester im Alkoholrest 1 bis 8 C-Atome enthalt.

3. Verfahren nach einem der Ansprüche 1 bis 2, **dadurch gekennzeichnet**, daß als Alkalialkoholat Natriummethylat und/oder Natriumethylat eingesetzt werden.

4. Verfahren nach mindestens einem der Ansprüche 1 bis 3, **dadurch gekennzeichnet**, daß pro mol Alkalialkoholat 1 bis 12 mol, vorzugsweise 1,5 bis 6 mol, Acetonitril eingesetzt werden.

5. Verfahren nach einem der Ansprüche 1 bis 4, **dadurch gekennzeichnet**, daß pro mol Alkalialkoholat 1 bis 2, vorzugsweise 1,5 bis 1,7 mol, Essigsäureester eingesetzt werden.

6. Verfahren nach mindestens einem der Ansprüche 1 bis 5, **dadurch gekennzeichnet**, daß Alkalialkoholat und Essigsäureester des gleichen Alkohols eingesetzt werden.

7. Verfahren nach mindestens einem der Ansprüche 1 bis 6, **dadurch gekennzeichnet**, daß die Umsetzung bei Temperaturen von 40 bis 160, vorzugsweise bei 80 bis 120 °C, unter Eigendruck oder bei erhöhtem Druck von 2 bis 200 bar durchgeführt wird.

8. Verfahren nach mindestens einem der Ansprüche 1 bis 7, **dadurch gekennzeichnet**; daß die Einsatzstoffe gemeinsam vorgelegt und zur Reaktion gebracht werden.

9. Verfahren nach mindestens einem der Ansprüche 1 bis 7, **dadurch gekennzeichnet**, daß der Essigsäureester in bis zu 10, vorzugsweise bis zu 2 Stunden, unter Reaktionsbedingungen einer Suspension von Alkalialkoholat in Acetonitril zugegeben wird.

10. Verfahren nach mindestens einem der Ansprüche 1 bis 7, **dadurch gekennzeichnet**, daß Essigsäureester und/oder eine Suspension von Alkalialkoholat in Acetonitril und/oder Acetonitril und Essigsäureester unter Reaktionsbedingungen dem Reaktionsgefäß portionsweise oder kontinuierlich zugeführt werden.

11. Verfahren nach mindestens einem der vorangehenden Ansprüche, **dadurch gekennzeichnet**, daß Gemische aus Essigestern und/oder aus mehreren Alkalialkoholaten eingesetzt werden mit untereinander verschiedenen Alkoholresten.

## Claims

1. A process for preparing alkali metal salts of cyanoacetone by reaction of acetonitrile and an acetic ester, the alcohol component of which is a substituted or unsubstituted, straight-chain, branched or cyclic alcohol, in the presence of alkali metal alkoxide, the alcohol component of which is a substituted or unsubstituted, straight-chain, branched or cyclic alcohol, at temperatures from 40 to 160°C, **characterized in that** the reaction is carried out without distilling off the alcohol formed during the reaction.

2. A process according to claim 1, **characterized in that** the acetic ester contains from 1 to 8 carbon atoms in the alcohol radical.

3. A process according to either of claims 1 and 2, **characterized in that** sodium methoxide and/or sodium ethoxide is/are used as alkali metal alkoxide.

4. A process according to at least one of claims 1 to 3, **characterized in that** from 1 to 12 mol, preferably from 1.5 to 6 mol, of acetonitrile are used per mole of alkali metal alkoxide.

5. A process according to at least one of claims 1 to 4, **characterized in that** from 1 to 2 mol, preferably from 1.5 to 1.7 mol, of acetic ester are used per mole of alkali metal alkoxide.

6. A process according to at least one of claims 1 to 5, characterized in that an alkali metal alkoxide and an acetic ester of the same alcohol are used.

7. A process according to at least one of claims 1 to 6, **characterized in that** the reaction is carried out at temperatures from 40 to 160°C, preferably from 80 to 120°C, under autogenous pressure or at an increased pressure of from 2 to 200 bar.

8. A process according to at least one of claims 1 to 7, **characterized in that** the starting materials are charged together and reacted.

9. A process according to at least one of claims 1 to 7, **characterized in that** the acetic ester is added over up to 10 hours, preferably up to 2 hours, under reaction conditions to a suspension of alkali metal alkoxide in acetonitrile.

10. A process according to at least one of claims 1 to 7, **characterized in that** the acetic ester and/or a suspension of alkali metal alkoxide in acetonitrile and/or acetonitrile and acetic ester are fed to the reactor in portions or continuously under reaction conditions.

11. A process according to at least one of the preceding claims, **characterized in that** mixtures of acetic esters and/or of a plurality of alkali metal alkoxides are used with each containing different alcohol radicals.

## Revendications

1. Procédé de fabrication de sels alcalins de la cyanacétone par réaction d'acétonitrile et d'un ester de l'acide acétique dont le composant alcool est un alcool substitué ou non substitué, à chaîne droite, ramifié ou cyclique en présence d'alcoolate de métal alcalin dont le composant alcool est un alcool substitué ou non substitué, à chaîne droite, ramifiée ou cyclique, à des températures de 40 à 160°C,
caractérisé en ce qu'
on conduit la réaction sans distillation de l'alcool formé au cours de la réaction.

2. Procédé selon la revendication 1,
caractérisé en ce que
l'ester de l'acide acétique dans le radical alcool contient de 1 à 8 atomes de carbone.

3. Procédé selon l'une des revendications 1 à 2,
caractérisé en ce qu'
on utilise comme alcoolate de métal alcalin le méthylate de sodium ou l'éthylate de sodium.

4. Procédé selon au moins une des revendications 1 à 3,
caractérisé en ce qu'
on utilise par mole d'alcoolate de métal alcalin de 1 à 12 moles, de préférence de 1,6 à 6 moles, d'acétonitrile.

5. Procédé selon l'une des revendications 1 à 4,
caractérisé en ce qu'
on utilise par mole d'alcoolate de métal alcalin de 1 à 2, de préférence de 1,5 à 1,7 mole, d'ester de l'acide acétique.

6. Procédé selon au moins une des revendications 1 à 5,
caractérisé en ce qu'
on utilise l'alcoolate de métal alcalin et l'ester d'acide acétique du même alcool.

7. Procédé selon au moins une des revendications 1 à 6,
caractérisé en ce qu'
on conduit la réaction à des températures de 40 à 160, de préférence entre 80 et 120°C, sous une pression propre ou à une pression accrue de 2 à 200 bars.

8. Procédé selon au moins une des revendications 1 à 7,
caractérisé en ce qu'
on dispose les matières utilisées et en ce qu'on les fait réagir ensemble.

9. Procédé selon au moins l'une des revendications 1 à 7,
caractérisé en ce qu'
on ajoute l'ester de l'acide acétique en une durée allant jusqu'à 10 heures, de préférence jusqu'à 2 heures, dans des conditions réactionnelles d'une suspension d'alcoolate de métal alcalin dans l'acétonitrile.

10. Procédé selon au moins une des revendications 1 à 7,
caractérisé en ce qu'
on ajoute en plusieurs fois ou de façon continue au vase à réaction l'ester de l'acide acétique et/ou une suspension d'alcoolate de métal alcalin dans l'acétonitrile et/ou l'acétonitrile et l'ester de l'acide acétique dans les conditions de la réaction.

11. Procédé selon au moins un des revendications précédentes,
caractérisé en ce qu'
on utilise des mélanges d'esters de l'acide acétique et/ou de plusieurs alcoolates de métaux alcalins avec des restes alcools différents l'un de l'autre.
